# EUROPEAN PATENT SPECIFICATION

(11) **EP 1 416 959 B1**
(45) Date of publication and mention of the grant of the patent: **23.06.2010**
(21) Application number: 02754001.2
(22) Date of filing: 02.08.2002
(51) Int. Cl.: A61K 39/39, A61K 9/127, A61P 37/04, A61P 35/00

(54) **LIPOSOMES PREPARED FROM THE EXTRACTABLE LIPIDS FROM A MYCOBACTERIUM**
AUS MYCOBACTERIUM EXTRAHIERBAREN LIPIDE HERGESTELLETE LIPOSOMEN
LIPOSOMES FORMES A PARTIR DE LIPIDES EXTRACTIBLES DE MYCOBACTERIUM

(30) Priority: 03.08.2001 US 309512 P
(43) Date of publication of application: 12.05.2004
(73) Proprietor: NATIONAL RESEARCH COUNCIL OF CANADA, Ottawa, Ontario K1A 0R6 (CA)
(72) Inventor: SPROTT, G., Dennis, Orleans, Ontario K4A 1Z6 (CA); KRISHNAN, Lakshmi, Gloucester, Ontario K1C 7M9 (CA); SAD, Subash, Gloucester, Ontario K1C 7M9 (CA)
(74) Representative: Rutherford, Claire
(86) International application number: PCT/CA2002/001217
(87) International publication number: WO 2003/011336

(56) References cited:
- EP-A- 0 241 376
- WO-A-01/26683
- SPROTT G ET AL: "Cellular immuno-stimulating potential of liposomes prepared from the polar lipids of Archaea and Bacteria." ABSTRACTS OF THE GENERAL MEETING OF THE AMERICAN SOCIETY FOR, vol. 101, 2001, page 349 XP008014632 101st General Meeting of the American Society for Microbiology;Orlando, FL, USA; May 20-24, 2001, http://www.asmusa.org/mtgsrc/generalmeetin g.htm 2001 ISSN: 1060-2011
- TENU JEAN-PIERRE ET AL: "Phosphatidylinositolmannoside-based liposomes induce no synthase in primed mouse peritoneal macrophages." BIOCHEMICAL AND BIOPHYSICAL RESEARCH COMMUNICATIONS, vol. 208, no. 1, 1995, pages 295-301, XP002234427 ISSN: 0006-291X

## Description

### FIELD OF THE INVENTION

This invention relates to the use of the chloroform-extractable polar lipids from the human vaccine strain of *Mycobacterium bovis* BCG, and other *Mycobacteria* with similar lipids, to prepare liposomes with immunomodulatory and adjuvant activity to promote an immune response to an associated antigen. Total polar lipids of BCG or purified lipid fractions PI, (phosphatidylinositol), PIM₁ (phosphatidylinositol mannoside), PIM₂ (phosphatidylinositol dimannoside) and their palmitoylated forms, or acylated-phospholipids of 899, 1139 and 1155 m/z are used to form liposomes at elevated temperatures and to activate antigen presenting cells in specific ways. The invention more specifically relates to vaccine development by providing a stable vehicle for antigen delivery to antigen presenting cells using immunostimulatory, chloroform extractable, polar BCG glycerolipids, resulting in enhancement of MHC class I and class II responses in an animal.

### BACKGROUND OF THE INVENTION

Historically, human vaccines have consisted of live attenuated viral or bacterial pathogens. Patient acceptance and safety is a concern based on possible side-reactions of complex and ill-defined vaccines, and the possibility for reversion to virulence. A more current approach is to use defined, highly purified antigens. Side-reactions are minimized, but the efficacy of these subunit vaccines is generally poor because of a loss in immunogenicity when the antigen is purified. A further difficulty is efficiently targeting both the antigen and adjuvant to precisely the same antigen presenting cells. Further, the lack of efficacy may be explained by an inappropriate immune response, because protection may require that either humoral, cell-mediated or cytotoxic T cell (CTL) responses predominate depending on the pathogen in question. For example, protective immunity against anthrax is thought to require only a humoral response, whereas, a protective vaccine against intracellular pathogens such as *M. tuberculosis* or cancers require a strong CTL response. The use of Alum as an adjuvant (approved for human use) is based on forming a complex with antigen to give a depot effect, resulting in only a Th2 response, and not CTL. Further, local reactions may occur at the injection site with aluminum-based adjuvants such as Alum (Koike *et al.* 1998).

Other adjuvant systems such as archaeosomes (Krishnan *et al.* 2001) and immunostimulating complexes (ISCOMS) are especially suited as cell-mediated adjuvants, but give only moderate antibody responses. ISCOMS have issues related to toxicity of saponin preparations used in their construction, and in use with water-soluble antigens (Bowersock and Martin 1999). In cases where the antigen and adjuvant are not co-delivered as a particulate system, inefficiency occurs in antigen delivery to the same antigen presenting cells activated by the adjuvant. Many delivery systems also require co-adjuvants such as Quil A or Lipid A (ex. ISCOMS, conventional liposomes) with expense, stability, and toxicity issues associated with their use. Ease of production and costs can be an issue for many of these adjuvant systems. Finally, lack of retention of the encapsulated antigen will make a vaccine vesicle system ineffective.

*Mycobacteria* spp. are often associated with pathogenesis and are best known as causative agents for tuberculosis (*M. tuberculosis),* leprosy (*M. leprae*), and as opportunistic pathogens (*M. avium*). The ability of the immune system to respond to mycobacterial cells, or their components, has been an area of keen interest for decades because of the pathogenicity associated with this genus.

The current vaccine against tuberculosis in humans is the culture of *Mycobacterium bovis* bacillus Calmette-Guérin (BCG) that became attenuated during passage in laboratory medium. Although the exact reasons for attenuation are still being researched (Behr *et al.* 2000), it was discovered early that heat-killed whole cells of *Mycobacterium tuberculosis* mixed with oil and an antigen resulted in strong adjuvant activity. This became known as Freund's Complete adjuvant (FCA) and has been used in many laboratories to promote a strong antibody, as well as a strong cytotoxic T cell (CTL) response (Skinner *et al.* 2001) to a protein antigen. Active components in FCA include muramyl dipeptide and trehalose 6,6'-dimycolate from the cell wall (Retzinger *et al*. 1981). Freund's Complete adjuvant is toxic causing acute inflammation, granulomas, and chronic toxicity (Retzinger *et al.* 1981) and is unacceptable, therefore, for human or veterinary use. The *Mycobacterium* spp. surface is composed of the cytoplasmic membrane surrounded by a cell wall made of mycoloyl arabinogalactan covalently attached to peptidoglycan, and associated lipoarabinomannan (LAM) (Chatterjee and Khoo 1998). All strains have these layers although the outer layer appears to differ in structural detail among strains (Ortalo-Magné *et al.* 1996). Lipids comprise part of these various outer layers and account for up to 60% by weight of the mycobacterial cell wall. This includes the mycolyl-arabinogalactan-peptidoglycan, covalently linked polymer, and several types of "extractable"lipids. "Extractable" lipids found in various strains include: (1) trehalose-containing glycolipids, (2) glycopeptidolipids, (3) phenolic glycolipids, (4) lipooligosaccharides, (5) phosphatidylinositol mannosides (PIMs), (6) phosphatidylethanolamine, and (7) triacylglycerols (Wang *et al.* 2000; Besra and Brennan 1994). The completed structures for novel palmitoyl and dipalmitoyl-PIMs has been reported only recently (Gilleron *et al.* 2001). Further, these authors showed that phosphatidylinositol (PI) had the same ability as PIM₁ and PIM₂ (all apparently adsorbed on Alum) to induce recruitment of Natural Killer T cells, indicating no difference in biological response with addition of mannose residues to PI (Gilleron *et al.* 2001). This biological effect is in direct contrast to the stimulation of dendritic cells to secrete IL-12 by PIMs, and not PI, as shown in the current invention.

LAMs represent the mycobacterial counterpart to Gram-negative lipopolysaccharides. These lipids are composed of a phosphatidylinositol anchor, a mannan core, an arabinan domain, and also mannooligosaccharide caps in the case of ManLAMs (Chatterjee and Khoo 1998). LAMs exert their effects on the immune system in several ways. For example, LAM isolated from actively growing mycobacteria activated cells expressing a Toll-like receptor 2 (TLR2) in a TLR-dependent fashion, but LAM isolated from BCG could not (Means *et al.* 1999). LAM is a water-soluble polymer and would not, therefore, be a component of the chloroform-soluble lipids used herein (Nigou *et al.* 1997).

Several other lipids of mycobacteria have immunomodulatory activity. The phenolic glycolipid trehalose 6,6'-dimycolate (cord factor) is an active component in FCA capable of promoting an antigen-specific CTL response (Skinner *et al.* 2001), and moderate antibody titres when injected with an antigen (Koike *et al.* 1998). This contrasts to the current invention in 1) liposomes were not used 2) antibody titres were not high with cord factor and 3) cord factor was absent from the lipids used in this invention.

Both the phenolic glycolipids and glycopeptidolipids have been shown to be on the cell surface and to be capable of down-regulating the immune system of the host during infection (Ortalo-Magné *et al* 1996). This down-regulation by surface lipid accounts, in part, for the success mycobacterial pathogens enjoy in evading the normal host response to invasion. This again is contrary to, teaching away from the current invention.

Whereas, EP-A-0 241 376 discusses liposomes made from a mixture of PIMs; and liposomes from total polar lipids of *M.bovis,* are mentioned in the abstract of Sprott G. et al, Abstracts of the General Meeting of the American Society for Microbiology, Vol. 101, 2001, page 349 (XP008014632); and liposomes made from BCG lipid mixture of PIM₁₋₅ lipids + cholesterol are described in the abstract of Tenu Jean-Pierre et al, Biochemical and Biophysical Research Communications Vo1.28, No. 1, 1995, pages 295-301, these disclosures do not teach the present invention as defined in the claims hereinafter.

### SUMMARY OF THE INTENTION

Liposomes form at elevated temperatures above 55°C, and preferably 65°C to 75°C, from the chloroform-soluble total lipid fraction of *Mycobacterium bovis* bacillus Calmette-Guérin (BCG), and its purified lipid components. Total polar lipids were separated by thin layer chromatography into eight fractions and characterized by specific spray reagents and mass spectrometry. Dendritic cells exposed to BCG total polar lipid liposomes were activated to excrete inflammatory cytokines, whereas lipids from commercial sources were relatively inactive. Dendritic cell activating for IL-12 secretion was localized to the phosphatidylinositol mannosides with a mannose residue (PIM¹ and PIM²) and their acylated forms, and to novel BCG acylated-phospholipids having fast atom bombardment mass spectrometry peaks at m/z 899, 1139 and 1155. Indeed, activity was considerably higher in these purified BCG lipid liposomes than in the BCG total polar lipid liposomes. In contrast, BCG phosphatidylinositol activated dendritic cells to secret tumor necrosis factor (TNF) (in absence of mannose residues) at amounts higher than BCG total polar lipid liposomes. This stimulation depended on the presence of the fatty acyl chain, tuberculosteric acid, characteristic of mycobacterial lipids, as PI from soybean was without effect. Cardiolipid, and phosphatidylethanolamine formed a major portion of the BCG total polar lipids yet in purified form had low activating activities. Mice immunized with a protein antigen entrapped in BCG total polar lipid liposomes produced both MHC class I and class II responses. Similar trials with liposomes composed of the total lipids extracted from another Gram-positive bacterium, *Bacillus firmus,* or PC/PG/cholesterol revealed that BCG liposomes had much superior adjuvant properties. A vaccine prepared from BCG liposomes gave protection to mice upon challenge with tumor cells.

According to one aspect of the present invention there is provided a liposome comprising one or more lipids obtainable from mycobacterium species, and selected from phosphotidylinositol (PI), phosphotidylinositol mannoside (PIM₁), phosphotidylinositol dimannoside (PIM₂), mono and dipalitoylated forms of PIM₁ and PIM₂, acylated-phospholipids having fast atom bombardment mass spectrometry peaks at 899, 1139 and 1155m/z, phosphotidylethanolamine and cardiolipid, wherein said lipids that are obtainable from mycobacterium are chloroform soluble and extractable.

Preferably the mycobacterium is *Mycobacterium bovis BCG.* The chloroform soluble and extractable lipid may be PIM₁, PIM₂, palmitoyl-PIM₁, palmitoyl-PIM₂ and preferably it is obtainable by 50% ethanol extraction.

The liposome may further comprise at least one other lipid which may be phosphatidylcholine, phosphatidylglycerol, cholesterol, or a mixture thereof.

According to another aspect of the invention there is provided a liposome vaccine composition comprising the liposome of the first aspect, where the liposome contains an associated antigen. The liposome vaccine may also comprise an antigen which may be a protein.

According to a further aspect of the present invention there is provided a method for preparing a liposome according to the first aspect, which method comprises drying chloroform soluble and extractable lipid from Mycobacterium SPP and then hydrating said dried lipid at t a temperature of 65 to 75°C in water or phosphate buffered saline (PBS).

A yet further aspect of the present invention relates to the use of a liposome according to the first aspect, in the manufacture of a medicament for stimulating an immune response in a mammal. Additionally there are further aspects of the present invention which relate to the use of the abovementioned liposome vaccine composition in preparing a medicament for the therapeutic or prophylactic treatment of cancer or in preparing a medicament to direct an immune response to confer protection against a pathogen or a cancer.

### BREIF DESCRIPTION OF THE DRAWINGS

Figure 1 is a fast atom bombardment mass spectrometer (FAB MS) spectrum of the chloroform extractable, total polar lipids of BCG. Signals are identified as PI (phosphatidylinositol); PE (phosphatidylethanolamine); PIM₁ (phosphatidylinositol) monomannoside); PIM₂ (phosphatidylinositol dimannoside); and fragment ion [PA] (phosphatidic acid). Assignments are shown in the figure for the total number of carbon atoms in the sn-1,2 glycerolipid fatty acyl chains: followed by number of unsaturations.

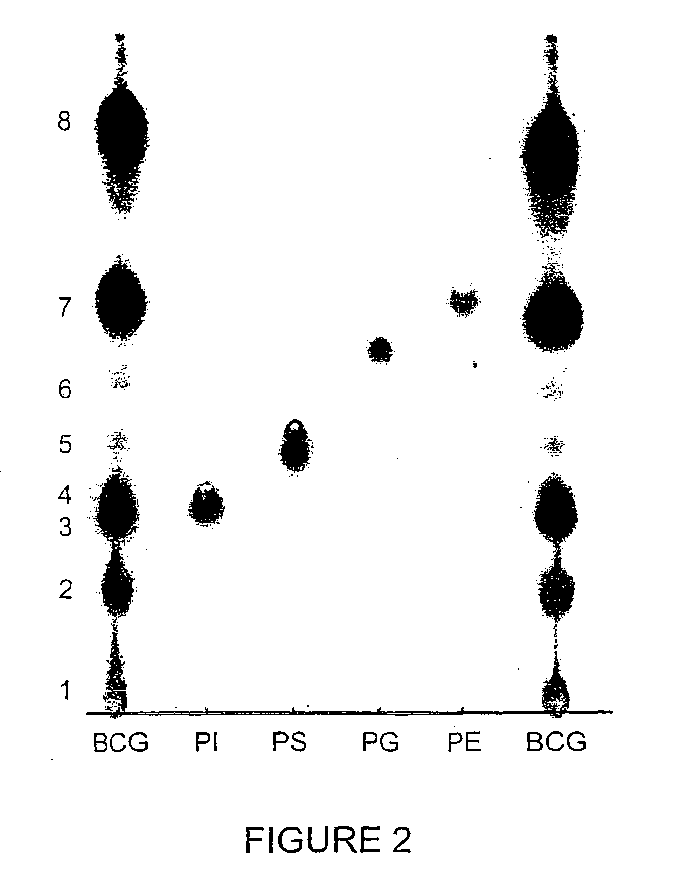

Figure 2 shows the separation of BCG total polar lipid into 8 lipid fractions by thin layer chromatography. Fractions are numbered 1 to 8, from most polar to least polar. Lipids (applied at the bottom of the plate) are BCG (chloroform extractable, total polar lipids), and PI, PS (phosphatidylserine), PG (phosphatidylglycerol), and PE (phosphatidylethanolamine) are reference standards. An acidic solvent was used to develop the plate. Lipids were located by spraying with the Zinzade phosphate spray reagent, so the spots shown are all phospholipids.
Figure 3 is a FAB MS spectrum of lipid fraction 1 showing that it contains saturated PIM₂ and a lipid of m/z 899. As shown, both lipids yield typical PA fragmentation to generate m/z 688+H, wherein the sum of carbon atoms in both sn-1,2 chains and their state of saturation is C35:0. The lipid structure of 899 m/z is shown to be a phosphatidylglycerol phosphate (PGP) acylated with a moiety of m/z 57.
Figure 4 is a FAB MS spectrum of lipid fraction 2 showing that it contains saturated palmitoyl-PIM₂ (Palm- PIM₂). Smaller amounts corresponding to about 12% PIM₁ and palmitoyl- PIM₁ are present also.
Figure 5 is a FAB MS spectrum of lipid fraction 3 showing that it contains saturated pure PI. About 80% of PI has C19:0 and C16:0 chains and much of the remaining PI has C19:0 and C15:0 chains.
Figure 6 is a FAB MS spectrum of lipid fraction 4 showing that it contains saturated dipalmitoyl-PIM₂ with sn-1,2 C19:0 plus C19:0 or C19:0 plus C16:0 glycerolipid chains. Some PI is found in this fraction also.
Figure 7 is a FAB MS spectrum of lipid fraction 5 showing that it contains saturated palmitoyl- PIM₁, PI, and about 12% palmitoyl-PIM₂.

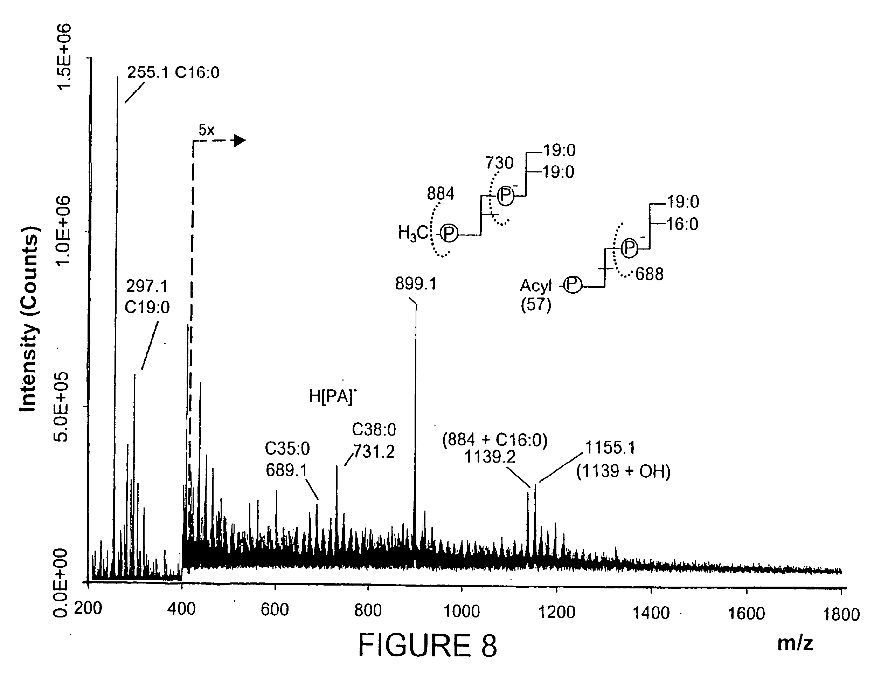

Figure 8 is a FAB MS spectrum of lipid fraction 6 showing that it contains saturated novel lipids of m/z 899.1, shown as acyl-PGPs (acyl-phosphatidylglycerol phosphate). In these PGP lipids the terminal phosphate is protonated, as indicated in the structure by a negative charge on only the phosphate closest to the glycerol backbone. A longer acyl-chain form of 1139.2 m/z corresponds to loss of the methyl group (on the phosphate) in the structure shown and replacement by a palmitoyl chain. Another related structure is 16 larger than 1139 indicating hydroxylation of one of the three acyl-chains.
Figure 9 is a FAB MS spectrum of lipid fraction 7 showing that it consists of saturated pure PE lipids of various chain lengths.
Figure 10 is a FAB MS spectrum of fraction 8 showing that it consists of unsaturated cardiolipid.
Figure 11 is a FAB MS spectrum of the total polar lipids extracted from *Bacillus firmus* and defines these lipids as primarily phosphatidylglycerols and cardiolipids.
Figure 12 shows that mice immunized subcutaneously at 0 and 3 weeks with antigen (OVA) entrapped in BCG liposomes provides protection against challenge with EG.7 (OVA expressing) tumor cells. It further shows that antigen-free BCG liposomes exert some innate protective effect against tumor growth seen as a delay in the onset of tumors.

### DETAILED DESCRIPTION OF THE INVENTION

Liposomes are closed spherical vesicles composed of a lipid bilayer with polar headgroups exposed to inner and outer surfaces and the lipid chains forming the interior part of the bilayer. Water-soluble drugs or antigens are either bound to the surface or entrapped in the fluid space within the liposome, whereas hydrophobic molecules tend to associate with the lipid layer.

In the present invention the total lipids from fresh BCG cells are extracted with ambient temperature methanol/chloroform/water (2:1:0.8, v/v), and the polar chloroform-extractable lipids separated from neutral lipids as the cold-acetone insoluble fraction. We show for the first time that this total polar lipid fraction, and purified lipids therefrom such as PIMs and palmitoyl-PIMs, will form liposomes provided the temperature is sufficiently high and preferably 65-75 °C. Animals may then immunized with BCG liposomes associated with one or more protective antigens to confer protection to pathogens or cancer where a strong immune response is required, or for the production of high antibody titres for research purposes. Adjuvant activity includes not only the MHC class II mediated Th2 and antibody arm of the immune response, but also MHC class I responses evident by induction of CTL responses and INF-gamma secreting CDS+ T cells in the Elispot assay and by showing that a BCG liposome vaccine can protect in a mouse tumor model. There have been no previous reports to our knowledge demonstrating either liposome construction from the lipids of mycobacteria, and specifically from the polar-extractable lipids, or reports of immunomodulation by such liposomes, thus obviating the need to include additional adjuvants in a BCG liposome vaccine.

Because temperatures above 55°C are required to prepare liposomes efficiently from BCG total polar lipids, or its purified components, it follows that at lower temperatures such as body temperatures the liposome membranes would pass from liquid crystalline phase to solid phase. In solid phase the membranes would be less leaky to entrapped antigen and stability of the liposomes would be enhanced resulting in an improved vaccine carrier. Further, several of the purified BCG lipids when converted to liposomes have immunostimulatory activity, shown herein by the activation of dendritic cells, the most potent cell type for processing and presenting antigen to T cells. The active lipids are composed of a glycerol backbone linked sn-1,2 with saturated fatty acyl C19:0 chains of tuberculosteric (unique to mycobacteria) and C16:0 palmitic acids, and a phosphoinositol headgroup at the sn-3 position linked to 1 or 2 mannose sugar residues (PIM1, PIM2), sometimes mono- or dipalmitoylated.

These active lipids may be obtained from BCG cells that are grown easily with high yield, or alternatively it may be appreciated they may be chemically synthesised, as their structures are known. Further, the tuberculosteric acid found in *Mycobacteria* species is a methyl-branched, long-chain, fully saturated fatty acyl chain, and as such is predicted to contribute to liposome stability. Indeed, the only BCG lipid that has unsaturation is the cardiolipids fraction S.

### MATERIALS AND METHODS

### Source of bacteria and growth

*Mycobacterium bovis* (BCG) Pasteur strain was obtained from Dr. Robert North (Trudeau Institute, USA) and grown aerobically in 1-liter shake flasks containing standard complex medium. *Bacillus firmus* was purchased from the American Type Culture Collection (ATTC 14575) and grown aerobically on Nutrient Broth 8 g/l, Yeast Extract 3 g/l (Difco Laboratories, Mich.), urea 1.5 g/l and Bactopeptone 1 g/l at 30 °C.

### Source of cell cultures

EL-4 and EG.7, a subclone of EL-4 stably transfected with the OVA gene, were obtained from the ATTC, and maintained and grown as described before (Krishnan *et al.* 2000).

### Lipid extraction

Briefly, total lipid extracts were obtained from frozen-thawed cell pastes of *B*. *firmus,* or from fresh cell paste of *M. bovis* by adding a one-phase solution of methanol, chloroform, and water (2:1:0.8, v/v) in a ratio of 15 g cell dry weight/1. After 16 h the cellular debris was collected by centrifugation and re-extracted twice more. Extracts were pooled and made biphasic by addition of chloroform and water by the Bligh and Dyer method previously described (Sprott *et al.* 1995). Polar lipids in the chloroform bottom phase were freed of neutral lipids by differential solubility in cold acetone (Sprott et al. 1995). Polar lipids, insoluble in cold acetone, were dried and dissolved into chloroform as the chloroform-extractable total polar lipids. BCG total polar lipids dissolved in chloroform were filtered using a 0.45 µm nylon syringe-filter, to ensure there was no carry over of whole cells into the lipid extract.

### Liposome preparation

1) *B. subtilis* liposomes - about 30 mg of total polar lipids in chloroform were dried under a nitrogen stream, and hydrated by adding 3.0-ml of pyrogen-free water. Hydration was allowed to proceed for 2-3 h at 35 °C with shaking prior to the addition of 10 mg ovalbumin (OVA)/30 mg lipid. Average vesicle diameters were decreased from 80 to100 nm in a sonic bath. Preparations were then freeze-dried and re-hydrated in phosphate buffered saline (PBS, 10 mM potassium phosphate plus 160 mM NaCl, pH 7.1). OVA was removed by centrifugation and three washes with PBS. The final liposome pellets were re-suspended into PBS, and liposomes filter-sterilized using syringe-driven 0.45 µm filters (Millipore, MA). Entrapped OVA was quantified after lipid removal by the SDS-Lowry colour development method as described before (Krishnan *et al.* 2000) and dry weights determined. Pyrogen-free sterile water was used throughout.
2) Commercial lipids - L-α-dimyristoylphosphatidylcholine (DMPC), L-α-dimyristoylphosphatidylglycerol (DMPG), L-α-phosphatidylinositol (soybean, PI), cardiolipid (bovine heart), and L-α-dipalmitoylphosphatidylethanolamine (DPPE) were purchased from Sigma. Liposomes were prepared as described for total polar lipids ofB. *subtilis,* except for the omission of OVA.
3) BCG liposomes - about 30 mg of total polar lipids in chloroform were dried under a nitrogen stream followed by 1-h under vacuum. Hydration was routinely done by adding 3-ml of pyrogen-free water containing the antigen (for example 10 mg OVA) and incubating for 2-3 h at 65°C. with shaking. To investigate the effect of temperature on liposome formation, hydration was allowed to occur at 3°C to 75°C, in 10°C steps. Average vesicle diameters were decreased between 80 - 100 nm in a sonic bath at 65°C. Preparations were then freeze-dried and re-hydrated in PBS at 65°C. Liposomes were left overnight at 4°C to anneal, then any OVA not associated with the liposomes was removed by ultracentrifugation and washing liposomes with PBS thrice. The final liposome pellets were re-suspended into PBS, and liposomes filter-sterilized using 0.45 µm filters. Entrapped OVA was quantified after lipid removal and dry weights determined, as above. Average diameters were measured in a 5 mW He/Ne laser particle sizer (Nicomp Model 370). BCG liposomes were made from isolated lipid fractions 1 to 8 by the above method, except for BCG PE fraction 7. PE liposomes were made by including 80 mole % DMPC, as PE lipids in general do not make liposomes in pure form. This also was the case for BCG PE.

### Lipid analysis

Polar lipid extracts were analysed by fast atom bombardment mass spectrometry (FAB MS) with a JEOLJMS-AX 505H instrument operated at 3 kV in negative ion mode. The xenon gun was operated at 10 kV. Current-controlled scans were acquired at a rate of 10-s full scale. A mixture of triethanolamine and Kryptofix® (Sigma) was used as the matrix. Staining for functional groups was done after separating the polar lipids on precoated 0.25 nun silica gel 60 thin-layer plates (Merck) developed with an acidic solvent chloroform/methanol/acetic acid/water (85:22.5:10:4, v/v) or basic solvent chloroform/methanol/7 N ammonium hydroxide (60:35:8, v/v). Lipid spots were characterized using the phospholipid (Zinzade's reagent), glycolipid (α-naphthol), aminolipid (ninhydrin), and total lipid (sulfuric acid char reagent) sprays described in Kates (1986). For sugar analysis lipids were first hydrolysed with 2 M trifluoroacetic acid for 2 h at 100°C. D-ribose was then added as an internal standard, and alditol acetate derivatives prepared for identification and quantification by gas chromatography-mass spectrometry (GC MS) (17). The total carbohydrate content of each lipid extract was determined by Anthrone reaction using D-glucose as the standard.

### Dendritic Cell (DC) isolation and activation

Bone marrow derived dendritic cells were prepared as described before (Krishnan *et al.* 2001), and were consistently > 80% CD11c⁺ by flow cytometry. Briefly, bone marrow was flushed from the femurs and tibias of C57BL/6 mice, and single cell suspensions made. Cells obtained were cultured (1X10⁶/ml) in RMPI medium supplemented with 8 % fetal bovine serum, FBS (RS) and 5 ng/ml of recombinant murine GM-CSF (ID Labs, London, ON, Canada) for 6-8 days at 37°C in 8% CO₂. Non-adherent cells were removed at days 2 and 4 of culture, and fresh R8 plus GM-CSF was added. Dendritic cells were harvested on days 6-8 as non-adherent cells. Dendritic cells (10⁵) were incubated *in vitro* with various concentrations of antigen-free archaeosomes or lipopolysaccharide (LPS, *E. coli,* Sigma), in triplicate in 96-well tissue culture plates, for 72 h at 37°C, 8 % CO₂, in a humidified atmosphere. At 72 h, activation of the cells was assessed by measurement of MTT (dimethylthioazol diphenyltetrazolium bromide) uptake and the supernatants were collected and IL-12 was assayed by sandwich ELISA (Mosmann & Fong 1989). TNF was assayed by a bioassay referenced in Krishnan *et al.* 2001.

### Immunizations

Female, C57BL/6 mice, 6-8 weeks of age, were immunized subcutaneously at the base of the tail at 0 and 21 days. Immunizations were with 15 µg OVA either with no adjuvant, or OVA entrapped in liposomes prepared from the total polar lipids extracted from BCG or *B. firmus.* In some cases FCA was included in the first immunization and Freund's incomplete adjuvant (FIA) in the second at 62% strength with 15 µg OVA in PBS.

### Analysis of humoral response

Sera were collected from blood obtained from the tail veins of mice and analysed for anti OVA antibodies. The antibody titres were determined by indirect antigen-specific ELISA. Briefly, ELISA plates (EIA microtitration plates, 96-well flat bottom, ICN Biomedicals Inc., Aurora, OH) were coated with antigen in PBS (10 µg/ml), and serial two-fold dilutions of serum (from individual mice) were assayed in duplicate. HRP-conjugated goat anti-mouse immunoglobulin (IgG + IgM) revealing antibody (Caltag, San Francisco, CA) was used to determine total antibody titres of sera. The reactions were developed with ABTS microwell peroxidase system (Kirkegaard and Perry Laboratories, Gaithesburg, Maryland) and absorbance determined at 415 nm after 15 min. Antibody titres are represented as endpoint dilutions exhibiting an optical density of 0.3 units above background.

### CTL assays

For CTL assays, 30 X 10⁶ spleen cells were cultured with 5 X 10⁵ irradiated (10,000 rads) EG.7 cells in 10 ml of RPMI plus 8 % FBS containing 0.1 ng/ml IL-2, in 25 cm² tissue culture flasks (Falcon), kept upright. After 5 days (37°C, 8% CO₂), the cells recovered from the flask were used as effectors in a standard ⁵¹Cr-release CTL assay and % specific lysis against EG.7 targets determined (Krishnan *et al.* 2001).

### ELISPOT assay

Enumeration of IFN-γ secreting cells was done by ELISPOT assay (Vijh and Pamer, 1997). Briefly, spleen cells were incubated in anti-IFN-γ antibody coated ELISPOT plates in various numbers (in a final cell density of 5x10⁵/well using feeder cells) in the presence of IL-2 (1 ng/ml) and RPMI media or OVA₂₅₇₋₂₆₄ (10 µg/ml) for 48 h at 37°C, 8 % CO₂. The plates were subsequently blocked, incubated with the biotinylated secondary antibody (4°C, overnight), followed by avidin-peroxidase conjugate (room temperature for 2 h). Spots were revealed using di-amino benzidine.

### Tumor model

A murine solid tumor model was used to assess the relative protective potential of CD8⁺ T cells induced by BCG TPL liposomes with OVA entrapped. Mice were injected twice at 0 and 21 days with OVA, 15 µg/0.1 ml injected per mouse, given subcutaneously. EG.7 cells (5 x 10⁶) expressing OVA (in PBS plus 0.5 % normal mouse serum) were injected in 0.1 ml in the shaved lower dorsal region, 9 weeks post first injection. From day 5 onwards, detectable solid tumors were measured using callipers. Tumor size, expressed in mm², was obtained by multiplication of diametrically perpendicular measurements.

### RESULTS AND DISCUSSION

### Lipid extraction from BCG

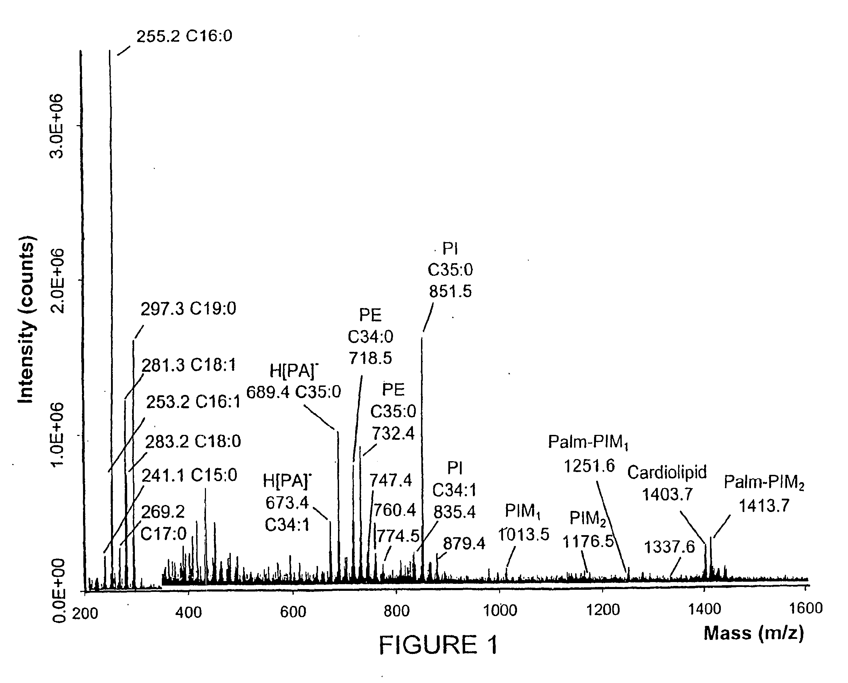

In this invention centrifuged cell pellets of BCG (10 g) are extracted at ambient temperature by stirring for 24 h with 1 liter of 1-phase Bligh and Dyer consisting of methanol/chloroform/water (2:1:0.8, v/v). The mixture is centrifuged at 10,500 x g for 15 min and supernatant and pellet fractions separated. The pellet fraction is extracted twice more as above and the three supernatants combined. Upon storing at 4°C a cloudy white precipitate forms and is removed by centrifuging at 4,100 x g for 15 min. This supernatant contains most of the chloroform extractable lipids. The small pellet removed is extracted again with 1-phase and the supernatant from this extraction combined with the chloroform extractable lipids. A volume of chloroform and water each equal to the total volume of the chloroform extractable lipids divided by 3.8 is added to obtain a 2-phase system in glass separatory funnels. The bottom chloroform phase containing the desired lipids is removed, and two 200-ml volumes of chloroform are used to wash the upper methanol-water phase. These chloroform washes are combined with the first chloroform phase. Also combined with the chloroform phases is chloroform recovered by centrifuging the milky emulsions formed at the interface of the two phases. Total polar lipids are recovered from the chloroform phases concentrated by flash evaporation by precipitating upon adding 20-volumes of ice-cold acetone. The pellet obtained is washed twice with ice cold acetone, dissolved in chloroform, and finally filtered through nylon 0.45 µm filters to obtain chloroform extractable total polar lipids (TPL). The total lipids account for about 10.2% of the starting BCG cell weight, of which 65% is total polar lipids and 35% acetone soluble lipids. A typical FAB MS spectrum of the total polar lipids is shown in figure 1. Dominant lipids were assigned as PE, PI, PIM₁, palmitoyl-PIM₁, PIM₂, palmitoyl- PIM₂, and cardiolipid. Dominant fatty acid carboxylate anions generated from the polar lipids during MS analysis are C16:0, C19:0 and C18:1. The signal of m/z 297.3 corresponds to the *M*. *tuberculosis* C19:0 fatty acid, 10-methyloctadecanoate, known as tuberculosteric acid (Leopold and Fisher 1993). A headgroup analysis shows mannose to be the major sugar present in about equal amount to inositol (Table 1).

A hot ethanol soluble lipid fraction may be obtained from the cell pellet above, already extracted three times with 1-phase Bligh and Dyer solution, by dispersing into 50% ethanol and refluxing at 65°C for 8 h. The mixture is centrifuged at 10,500 x g for 20 min and the supernatant flash evaporated to remove the ethanol. The remaining liquid is extracted with 1-phase Bligh and Dyer solution and made 2-phase to recover the hot ethanol lipids in the chlorofonn phase. Hot ethanol extracted lipids were similar to TPL in mannose and inositol content and represent roughly half of the mannose and inositol recovered in TPL lipids (Table 1). Thin layer chromatography and FAB MS show the same lipids present as in TPL. Thus, although only the chloroform extractable TPL is used herein, it is appreciated that TPL yield may be increased by combining, or replacing with a hot ethanol extraction. It may also be appreciated that a hot ethanol extraction may be used as an alternative to the Bligh and Dyer method to obtain essentially the same lipids using less costly and less toxic solvent.

### Purification and characterization of the BCG TPL lipids

Thin layer chromatography is used to separate TPL into 8 fractions, all of which stain positively for phosphate (Fig. 1). Fraction 4 has similar mobility to standard PI and fraction 7 corresponds to a PE standard. Staining reactions further define these 8 lipid fractions (Table 2). Lipids 1, 2, 4 and 5 are phosphoglycolipids, 3, 6 and 7 are phospholipids, and lipid 7 is a phosphoaminolipid.

To purify components of TPL, BCG TPL is applied as a band (6 mg/plate) and separated in this way into the 8 fractions by locating bands with iodine vapor and recovering the chloroform soluble lipids from the removed adsorbent. Bands 3 and 4 merge and may be recovered together, then separated and recovered using another thin layer plate and an alkaline solvent. The relative abundance of each recovered fraction 1 to S is shown in Table 2.

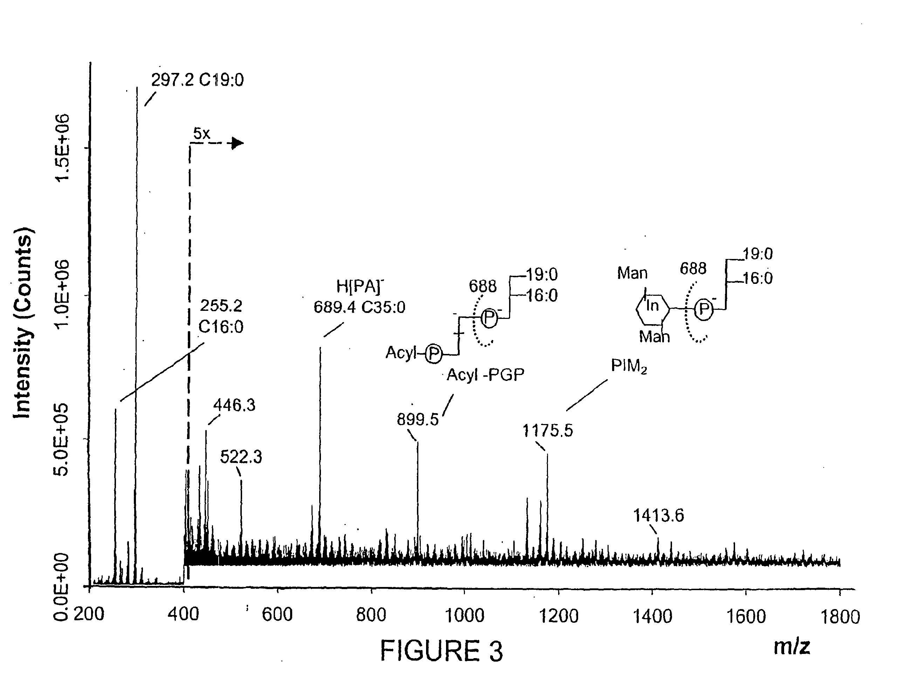

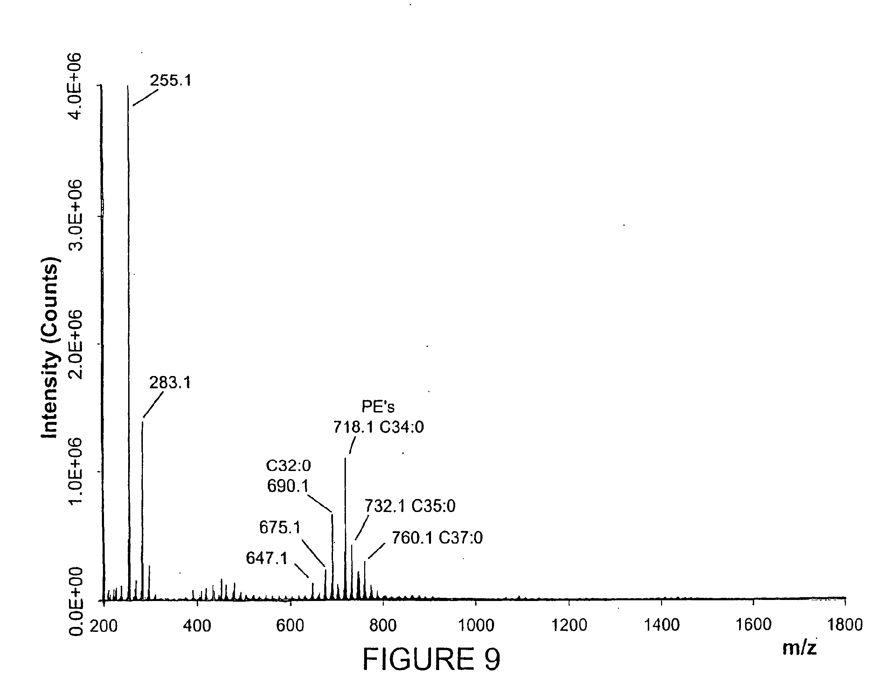

The 8 lipid fractions are defined structurally by FAB MS analysis in figures 3 to 10. Fraction 1 consists of PIM₂ and an 899.5 m/z lipid. Both have C19:0 and C16:0 chains as only these two chains are seen as carboxylate anions (Figure 3). The 899.5 m/z lipid is clearly in low relative amount, as it is not seen in a spectrum of TPL (Figure 1). An acyl-PGP lipid structure consistent with the above spectrum is shown in figure 3, in which the acyl group must be 57 m/z (either a propionic fatty acyl or butyl group). In these structures of BCG lipids, glycerol moieties are shown in 'stick' form and tuberculosteric acid chain position is sn-1 based on Gilleron *et al.* (2001).

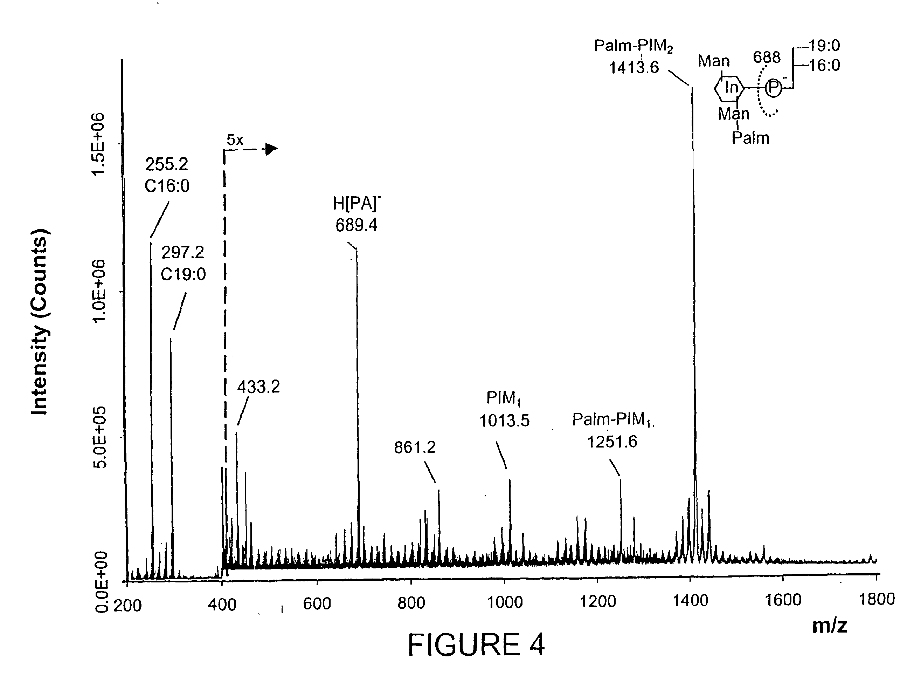

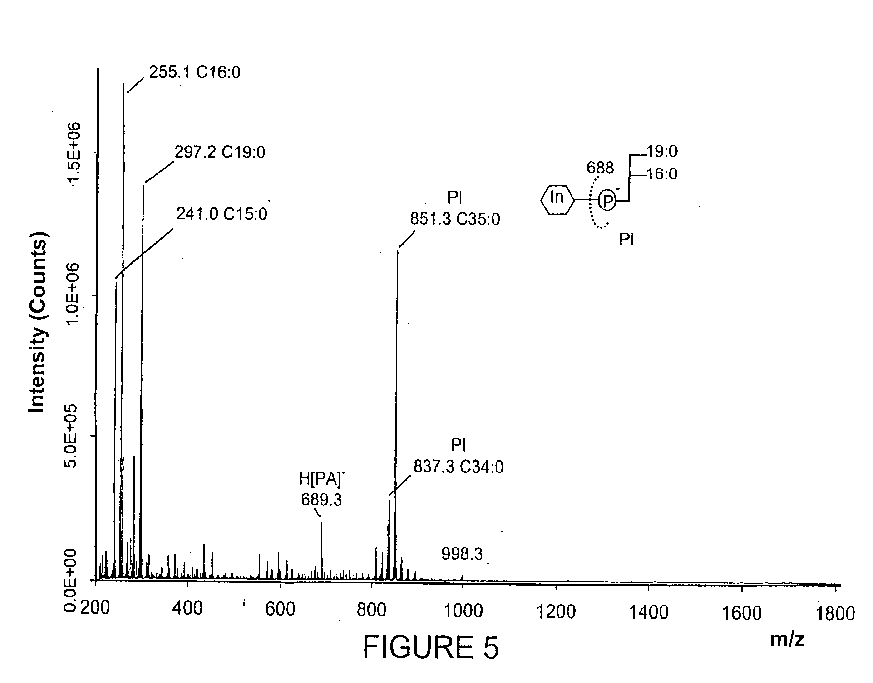

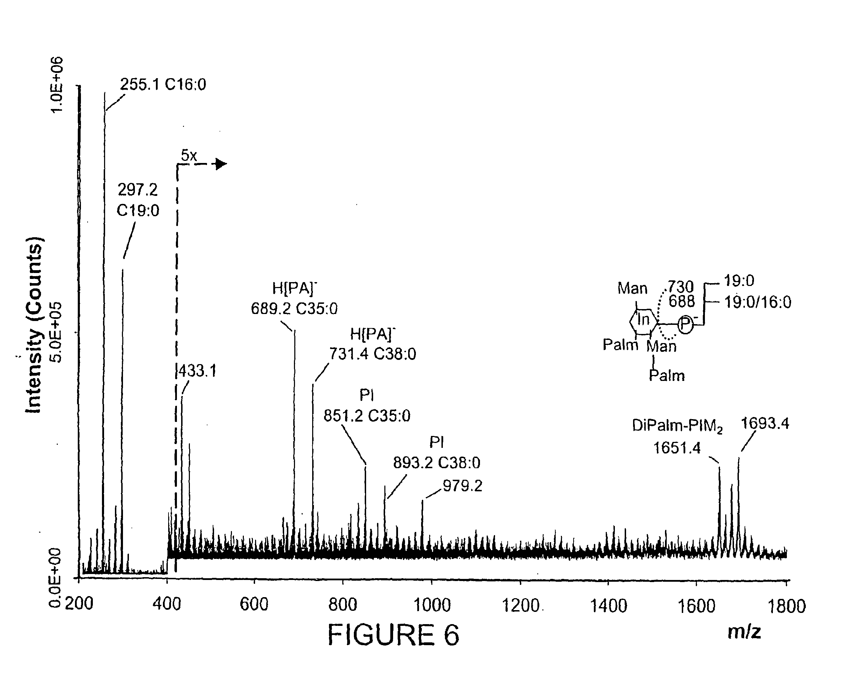

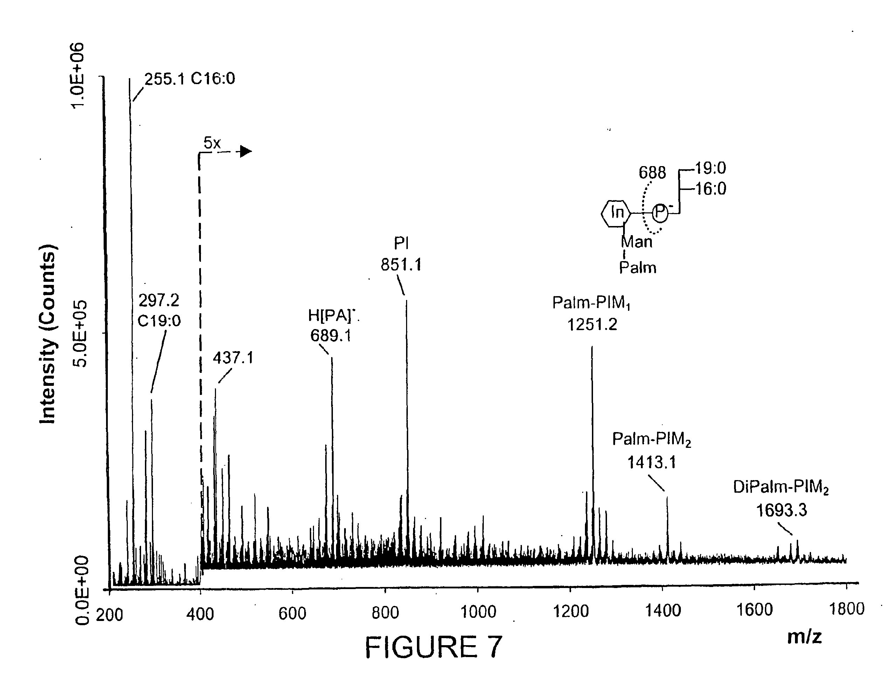

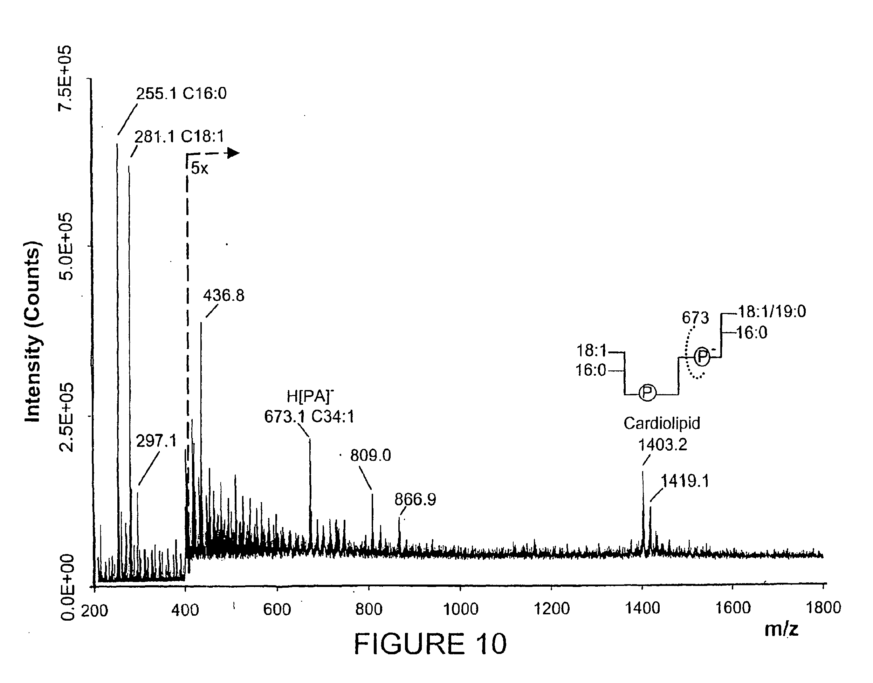

Lipid fraction 2 is primarily palmitoyl- PIM₂ with C19:0 and C16:0 chains (Figure 4), and fraction 3 is pure PI (Figure 5). In the case of PI most molecules have C19:0 plus C16:0 chains, with the bulk of the remaining PI having C19:0 and C 15:0 chains. Fraction 4 is a phosphoglycolipid defined by FAB MS as dipalmitoyl- PIM₂ of various sn-1,2 chain forms ranging from C35:0 to C38:0 (Figure 6). PI is detected also in this fraction. Palmitoyl- PIM₁ and PI in about equal amount comprise fraction 5 (Figure 7) a phosphoglycolipid fraction of only 3% abundance in BCG TPL (Table 2). Fraction 6 is a phospholipid of m/z 899.1 (Figure S) comprising only 1.2% of TPL. The most dominant PA fragment ion is 731.2 m/z indicating a methyl-PGP with sn-1,2 tuberculosteric acid fatty acid chains. However, other PA fragment anions, and fatty acid carboxylate signals, indicate PGP molecules with other sn-1,2-chains and acyl moieties on the terminal phosphate to total a m/z of 899.1, the primary signal for the molecular anion. Clearly, fraction 7 is pure PE with several sn-1,2 chain combinations, primarily C18:0 plus C16:0 (C34:0). PA fragment ions at m/z 647.1 and 675.1 confirm the PE assignments and correspond to fragments from C32:0 and C34:0, respectively. Finally, mobility of thin layer plates, staining reaction, and FAB MS identifies fraction 8 as a cardiolipid with mainly C18:1 and C16:0 chains and a molecular anion signal of 1403.2 m/z (Figure 10).

### Liposome formation from BCG chloroform extractable polar lipids

Contrary to expectation BCG TPL (total polar lipid) did not form liposomes at the normal growth temperature of *M. bovis* BCG, namely 37°C. TLP lipids were dried from solvent and liposomes monitored by phase microscopy after addition of water, or PBS buffer, at 35, 45, 55, 65, and 75°C. Liposomes did not form well at 55°C or less, resulting in clumps of lipid, but elevating the temperature to 65 or 75°C resulted in a dramatic formation especially when water was used for hydration. In this invention then, chloroform extractable BCG TPL is hydrated preferably at 65°C in the presence of antigen to form multilamellar liposomes. Smaller liposomes are produced, if desired, by size reduction at preferably 65°C using a bath sonicator. Other methods of size reduction could be used if the temperature is 65°C. Entrapment of antigen may be improved by lyophilization and rehydration of the liposome powder in water at 65°C, followed by PBS. Liposomes are then annealed and any unentrapped antigen removed as described in Materials and Methods. Average diameters were 230 ± 136 nm with OVA loadings in 3 preparations ranging from of 33 to 67 µg/mg dry weight of liposomes. Average diameters of BCG liposomes made from the purified lipid fractions are shown in Table 3. Those skilled in the art will appreciate that the various methods described in liposome formation should apply to these BCG lipids providing care is taken to achieve the required temperature, preferably 65°C.

### Activation of dendritic cells by BCG TPL liposomes and liposomes prepared from purified lipids fraction 1 to 8

Because dendritic cells represent the major antigen presenting cells in mammals, they are the preferred cells for *in vitro* adjuvant testing. Bone marrow dendritic cells were cultured with zero (R8 medium only) to 10 µg dry weight liposomes/ml of R8 medium. Liposomes tested are shown in table 3 and include several made from commercial lipids. After 72 h the numbers of viable cells were quantified by the MTT assay and secreted inflammatory cytokines assayed in the culture supernatants. Liposomes at 10 µg/ml giving an MTT of >25% above the control R8 medium, were limited to BCG TPL liposomes, and purified BCG lipid fractions 3 (PI) and 6. All liposomes made from non-BCG lipids; namely, DMPC, DMPG, PI from soybean, DPPE + DMPC, and cardiolipid from brain, were without significant activity, measured as IL-12 secretion from dendritic cells. However, PIM fractions 1 and 2, as well as fraction 6, induced secretion of IL-12 several-fold above that induced even by BCG TPL liposomes (at 10 µg/ml). Further, induction of IL-12 secretion required the addition of at least the complexity of 1 mannose unit to BCG PI, as purified BCG PI (fraction 3) was relatively inactive in this regard.

Further unexpected results are seen in the case of induction of TNF (tumor necrosis factor) secretion from dendritic cells. BCG TPL liposomes were again active. However, contrary to the effects on IL-12 secretion, TNF secretion occurred with purified BCG PI liposomes, and not with other BCG lipid liposomes or commercial lipid liposomes, clearly showing that BCG PI is the active lipid in BCG TPL accounting for TNF secretory activity. The fact that PI from soybean (with no tuberculosteric acid chains) was inactive, while BCG PI was highly active points solidly to the tuberculosteric acid (C19:0) as a key structural difference to explain active versus inactive PIs. Clearly, the lipids in BCG TPL have very different biological effects on activation and cytokine secretion from dendritic cells and consequently on the type of immune response obtained. Also clear, preparing liposomes using various combinations of BCG polar lipids is indicated as a mechanism to direct the type of immune response obtained to an entrapped antigen.

Phosphatidylethanolamines (PEs) are known generally as fusogenic lipids, capable of promoting fusion of membranes, and account for about 25% of the lipids in BCG TPL (Table 2). To mount a CTL immune reaction it is first necessary to deliver antigen to the cytosol of antigen presenting cells. It follows that inclusion of this lipid in a liposome with antigen entrapped, may aid in directing the immune response to MHC class I presentation of antigen and mounting a CTL response.

### Immune response in mice

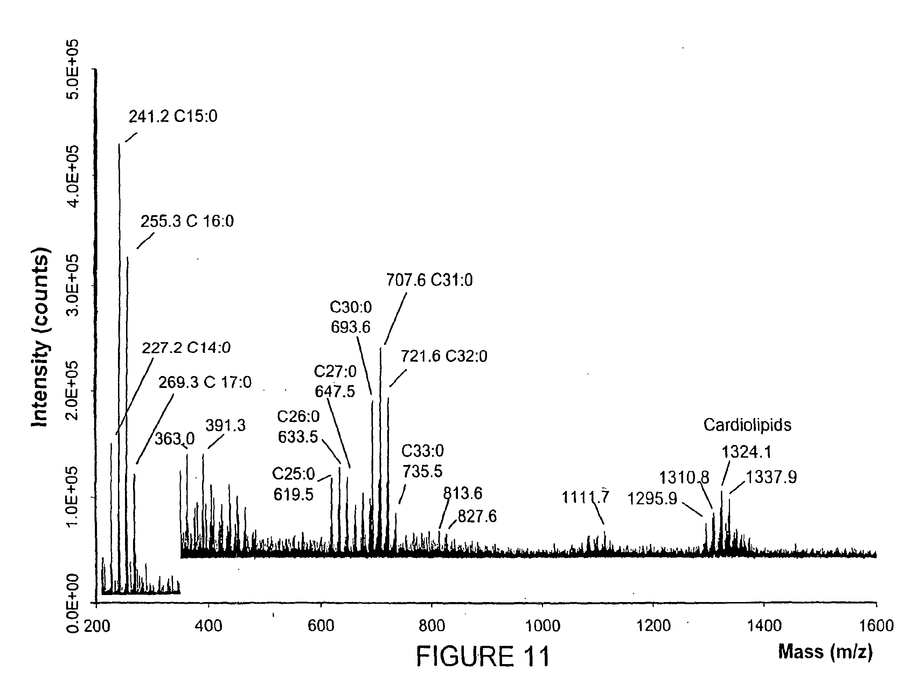

In a first example the adjuvant activity of BCG liposomes is compared to several other adjuvant systems. In one of these TPL liposomes from another Gram positive bacterium are included. The bacterium chosen was *Bacillus firmus* based on the observation that injections of these lipids into mice 5 days prior to infection with *Listeria monocytogenes* resulted in some short-term protection (Mára *et al.* 1992), presumably by activating the innate immune system. The TPL lipids of this bacterium extracted by the Bligh and Dyer method and collected as the acetone insoluble lipids, are characterized by FAB MS (Figure 11). In the case of *B. firmus* polar lipid extracts, m/z signals for the molecular anion of each lipid were assigned to a cluster of PG lipids with fully saturated sn-1,2 fatty acyl chains consisting of from 25 to 33 carbon atoms (sum of both chains) (Fig. 3). These assignments are consistent with the m/z of the carboxylate anions generated from the fatty acyl glycerochains during the analysis, which ranged from C13:0 to C17:0. Also, signals were found in the cardiolipid and LPG (lysophosphatidylglycerol) regions of the spectrum. An amino acid analysis confirmed the presence of small amounts of lysine in hydrolysates of *B. firmus* polar lipid extract, indicating the possibility of LPG and/or lysylcardiolipids (Fisher and Leopold 1999).

Table 4 represents a first example of an enhanced immune cytotoxic T cell (CTL) response raised in an animal to an antigen entrapped in BCG liposomes. BCG liposomes served to promote an immune response to the entrapped antigen that was similar to live BCG recombinant, and superior to Freund's adjuvant. Further, the TPL of another Gram positive bacterium also with saturated glycerolipids formed liposomes with inferior properties to BCG liposomes, teaching away from the positive result with BCG liposomes.

Table 5 describes the humoral adjuvant activity of BCG liposomes to entrapped protein. First, injection of equivalent amounts of BCG liposomes and OVA produced no adjuvant activity, whereas entrapment resulted in humoral adjuvant activity comparable to the adjuvant Alum. The toxic adjuvant FCA was superior in potency and in maintaining the antibody titre for longer periods after vaccination. Live recombinant BCG expressing OVA *in vivo* produced CTL as expected (Dudani et al, 2002), but no antibody response. Finally, liposomes containing no BCG lipids gave very low antibody titres that improved as BCG lipids were incorporated at increasing amounts from 10 to 50%.

The effect of loading BCG liposomes with different amounts of antigen per mg liposomes is shown to be insignificant from the range of 15 µg antigen loaded in 0.22 to 1.8 mg (dry weight) of liposomes. This is shown in Table 6 for the MHC class I-restricted, CD8⁺ T cell response. In A) results are shown of CTL assays and in B) numbers of IFN-gamma secreting precursor T cells in spleens of the variously immunized mice that specifically recognize the antigen in the original vaccination. Both assays show good adjuvant activity but no differences based on loading of the vaccine within this range.

### Antigen loaded BCG liposomes as protective vaccines

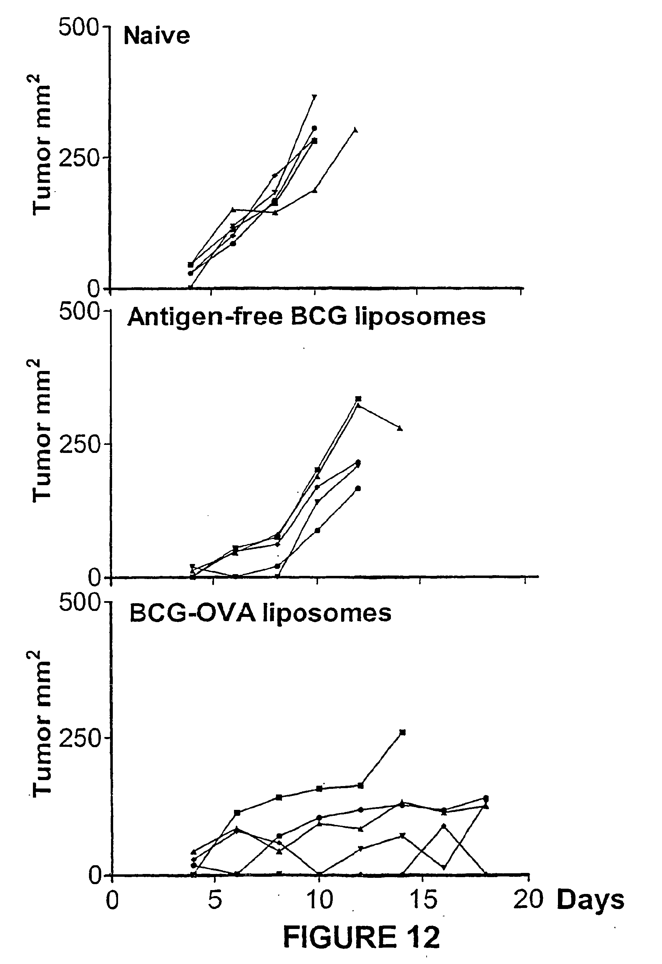

Mice given large numbers of EG.7 tumor cells develop rapidly growing solid tumors reaching >250 mm² in all 5 mice in the naïve group within 12 days (Figure 12). Injections of antigen-free BCG liposomes resulted in a modest decline in tumor growth, where only 2 mice out of 5 developed tumors >250 mm² after 12 days. Furthermore, a clear delay in the onset of tumor growth is seen. Considerably more protection was seen for mice immunized with the BCG OVA liposome vaccine, where in all mice tumors were <250 mm² after 12 days and remained so for the duration of the study (for 4 mice out of 5).

**Table 1. Percent relative abundance of inositol and mannose headgroups in BCG total polar lipids (TPL) and hot ethanol extracted lipids.**

| | | | |
|---|---|---|---|
| BCG cells were first extracted by the Bligh and Dyer method to generate TPL. These cells were extracted again with hot 50% ethanol to yield a hot ethanol lipids. | | | |

| | | TPL | Hot ethanol |
|---|---|---|---|
| % | carbohydrates | 5.62 | 2.79 |
| | Mannose | 4.52 | 1.96 |
| | Glucose | 1.05 | 0.73 |
| | Galactose | 0.0 | 0.0 |
| | Mannosamine | 0.0 | 0.0 |
| | Glucosamine | 0.0 | 0.0 |
| | Galactosamine | 0.0 | 0.0 |
| | Arabinose | 0.062 | 0.099 |
| Inositol (% of Man) | | 4.29 | 2.57 |

**Table 2. Thin layer chromatography of the chloroform extractable total polar lipids from BCG cells into 8 fractions.**

| | | | | |
|---|---|---|---|---|
| An acidic solvent was used to separate fractions 1, 2 and 5 to S. In the case of fractions 3 and 4 the lipids were recovered together and run again on a second thin layer plate using a basic solvent to achieve separation. Staining reactions and recoveries are shown for each lipid fraction. | | | | |

| Fraction | Phosphate stain | Sugar stain | Amino stain | % (w/w) abundance in TPL |
|---|---|---|---|---|
| 1 | + | + | - | 8.6 |
| 2 | + | + | - | 12.4 |
| 3 | + | - | - | 14.1 |
| 4 | + | + | - | 10.4 |
| 5 | + | + | - | 3.0 |
| 6 | + | - | - | 1.2 |
| 7 | + | - | + | 25.9 |
| 8 | + | - | - | 24.4 |

**Table 3. Activation of bone marrow dendritic cells by antigen-free BCG liposomes.**

| | | | | | | | | | |
|---|---|---|---|---|---|---|---|---|---|
| The ability of liposomes prepared from BCG total polar lipid and BCG lipid fractions 1 to 8 are compared to liposomes made from commercial lipids. Background measurements for RS medium alone were not subtracted from the values in the table, but were 0.542 (MTT), 9.3 (IL-12), and 0 (TNF). ND, not done. | | | | | | | | | |

| Liposome (diameter nm) | MTT (Absorbance) | | | IL-12 (ng/ml) | | | TNF (pg/ml) | | |
|---|---|---|---|---|---|---|---|---|---|
| | 0.1 µg/ml | 1 µg/ml | 10 µg/ml | 0.1 µg/ml | 1 µg/ml | 10 µg/ml | 0.1 µg/ml | 1 µg/ml | 10 µg/ml |
| BCG TPL | 0.418 | 0.495 | 0.727 | 10.8 | 8.5 | 48.5 | 19 | 45 | 26 |
| (414 ± 273) | | | | | | | | | |
| BCG1 | 0.374 | 0.321 | 0.574 | 4.2 | 3.0 | 168.4 | 0 | 1 | 0 |
| (53 ± 34) | | | | | | | | | |
| BCG2 | 0.468 | 0.415 | 0.618 | 4.5 | 29.8 | 230.2 | 1 | 1 | 10 |
| (48 ± 44) | | | | | | | | | |
| BCG3 | 0.448 | 0.382 | 0.774 | 2.5 | 2.8 | 36.6 | 46 | 80 | 146 |
| (53 ± 35) | | | | | | | | | |
| BCG5 | 0.459 | 0.532 | 0.613 | 4.0 | 13.8 | 92.9 | 0 | 0 | 0 |
| (162 ± 97) | | | | | | | | | |
| BCG 6 | 0.438 | 0.501 | 0.745 | 7.3 | 26.6 | 147.2 | 0 | 5 | 25 |
| (138 ± 111) | | | | | | | | | |
| BCG 7+DMPC | 0.407 | 0.443 | 0.499 | 7.2 | 8.6 | 39.9 | 0 | 0 | 0 |
| (68 ± 37) | | | | | | | | | |
| BCG8 | 0.439 | 0.604 | 0.896 . | 3.2 | 5.2 | 41.6 | 0 | 0 | 32 |
| (45 ± 24) | | | | | | | | | |
| DMPC | 0.431 | 0.438 | 0.394 | 9.0 | 7.9 | 13.1 | 0 | 4 | 2 |
| (270 ± 214) | | | | | | | | | |
| DMPG | 0.421 | 0.386 | 0.404 | 3.6 | 4.8 | 8.4 | ND | ND | ND |
| (94 ± 49) | | | | | | | | | |
| PIsoybean | 0.402 | 0.408 | 0.448 | 6.0 | 5.3 | 7.6 | 0 | 1 | 2 |
| (29 ± 20) | | | | | | | | | |
| DPPE + DMPC | 0.372 | 0.388 | 0.439 | 7.6 | 5.4 | 3.4 | 4 | 12 | 20 |
| (135) | | | | | | | | | |
| Cardiolipid | 0.419 | 0.429 | 0.609 | 6.8 | 6.6 | 10.2 | 11 | 14 | 5 |
| (128 ± 73) | | | | | | | | | |
| LPS | - | 0.564 | 0.562 | - | 187.9 | 182.7 | ND | 110 | 968 |

**Table 4. Comparison of CTL activity in splenic cell cultures from mice immunized with OVA in various adjuvant systems.**

| | | | | | |
|---|---|---|---|---|---|
| Mice were immunized subcutaneously at 0 and 21 days with 15 µg OVA entrapped in BCG total polar lipid liposomes, mixed with Freund's adjuvant, or entrapped in liposomes prepared from the total polar lipids extracted from *B. firmus* (92 ± 48 nm diameter, loading 53 µg/mg). In the case of live BCG cells expressing OVA, only one subcutaneous injection was given containing 10⁶ cells. Spleens from duplicate mice were pooled for each analysis 10 weeks post first injection with exception of *B. firmus* taken 6 weeks post first injection. Lysis of control EL-4 cells not expressing the OVA peptide was always <2%, and % lysis of target (T) EG.7 cells expressing OVA by splenic effector (E) cells is shown in the table. | | | | | |

| E:T ratio | Naïve | FCA | Live BCG | BCG liposomes | *B. firmus* liposomes |
|---|---|---|---|---|---|
| 100:1 | 1 ± 1 | 17 ± 1.8 | 39 ± 2 | 30 ± 2 | 11 ± 0.6 |
| 33:1 | 2 ± 3 | 9 ± 1.7 | 23 ± 0.9 | 15 ± 1.5 | 9 ± 4 |
| 11:1 | 0 ± 1 | 3 ± 0.5 | 11 ± 3 | 8 ± 0.9 | 2.5 ± 0.3 |
| 3.7:1 | 1 ± 0 | 0.6 ± 0.7 | 4 ± 0.9 | 4 ± 0.4 | 1 ± 0.4 |

**Table 5. Comparison of anti OVA antibody titres in sera of mice immunized with OVA in various adjuvant systems.**

| | | | |
|---|---|---|---|
| In all cases groups of 4 to 7 C57BL/6 mice were immunized with 15 µg OVA per injection at 0 and 3 weeks. BCG liposomes with OVA entrapped were prepared from 100% BCG TPL lipids mixed with DMPC/DMPG/cholesterol lipids from 0 to 100%, such that 0% BCG lipids were pure DMPC/DMPG/cholesterol liposomes. BCG OVA-free liposomes were separate injections of an equivalent amount of antigen-free BCG liposomes and OVA (unentrapped). For FCA OVA was mixed with FCA for the first injection and FIA for the second. Alum was Imject Alum to which OVA was bound. BCG live are BCG cells genetically modified to express OVA (see table 4 injection details). Blood was taken at various time points from first injection and anti OVA antibody in the sera was titrated by ELISA. | | | |

| Adjuvant | Day 10 | Day 31 | Day 41 |
|---|---|---|---|
| 100% BCG liposomes | 740 ± 380 | 23309 ± 16863 | 8266 ± 5240 |
| 50% BCG liposomes | 55 ± 54 | 14227 ± 4301 | 9643 ± 4287 |
| 10% BCG liposomes | 10 ± 0 | 1600 ± 1062 | 1499 ± 978 |
| 0% BCG liposomes | 10 ± 0 | 1065 ± 1020 | 857 ± 839 |
| BCG OVA-free liposomes | 0 | 388 ± 259 | 345 ± 350 |
| FCA | 5156 ± 6722 | 41420 ± 26376 | 65671 ± 26080 |
| Alum | 472 ± 371 | 8306 ± 2074 | 12302 ± 6848 |
| BCG live | 50 | 0 | 0 |
| no adjuvant | 0 | 519 ± 552 | 821 ± 738 |

**Table 6. Effect of antigen loading in BCG liposomes on induction of an immune response in mice.**

| 6 wk pfi | | | | |
|---|---|---|---|---|
| A. CTL - % lysis of EG.7 targets | | | | |
| E:T ratio | Naive | 15 µg/1.8 mg | 15 µg/1.6 mg | 15 µg/0.22 mg |
| 100:1 | 0.9 ± 0.1 | 52 ± 2 | 51 ± 2 | 55 ± 2 |
| 33:1 | 0 ± 0.7 | 41 ± 3 | 36 ± 3 | 45 ± 3 |
| 11:1 | 0 ± 1 | 29 ± 3 | 22 ± 3 | 31 ± 5 |
| 3.7:1 | 0 ± 0.9 | 16 ± 2 | 10 ± 0.1 | 15 ± 2 |

| B. Elispot - number of IFN secreting colonies | | | | |
|---|---|---|---|---|
| OVA peptide | Naive | 15 µg/1.8 mg | 15 µg/1.6 mg | 15 µg/0.22 mg |
| + | 0 | 23 ± 1 | 26 ± 3 | 24 ± 3 |
| - | 0 | 0 | 0 | 0 |

| C. anti OVA antibody titres (2 to 4 mice/group) | | | | |
|---|---|---|---|---|
| Mouse number | OVA, no adjuvant | 15 µg/1.8 mg | 15 µg/1.6 mg | 15 µg/0.22 mg |
| 1 | < 42 | 2922 | 2334 | 2965 |
| 2 | < 42 | 5552 | 1973 | 2346 |
| 3 | < 42 | - | 1692 | 3063 |
| 4 | < 42 | - | 3034 | 2797 |

### References

Behr, M.A., B.G. Schroeder, J. N. Brinkman, R.A. Slayden, and C.E Bany (2000) A point mutation in the mma3 gene is responsible for impaired methoxymycolic acid production in Mycobacterium bovis BCG strains obtained after 1927. J. Bacteriol. 182: 3394-3399.
Besra, G.S. and P.J. Brennan (1994) The glycolipids of mycobacteria. In: Mass spectrometry for characterization of microorganisms. American Chemical Society.
Bowersock, T.L. and S. Martin (1999) Vaccine delivery to animals. Advanced Drug Delivery Rev. 38: 167-194.
Chatterjee, D. and K.-H. Khoo (1998) Mycobacterial lipoarabinomannan: an extraordinary lipoheteroglycan with profound physiological effects. Glycobiology 8: 113-120.
Dudani, R. Chapedelaine, Y. van Fassen, H. Smith, D.K. Shen, H. Krishnna, L. Sad, S. (2002) Multiple mechanisms compensate to enhance tumor-protective CD8+ T cell response in the long-term despite poor CD8+ T cell priming initially: comparison between an acute versus a chronic intracellular bacterium expressing a model antigen. J. Immunol. 168:5737-5745.
Fischer, W. and K. Leopold (1999) Polar lipids of four listeria species containing L-lysylcardiolipin, a novel lipid structure, and other unique phospholipids. International Journal of Systematic Bacteriology 49: 653-662.
Gilleron, M., C. Ronet, M. Mempel, B. Monsarrat, G. Gachelin, and G. Puzo (2001) Acylation state of the phosphatidylinositol mannosides from Mycobacterium bovis Bacillus Calmette Guérin and ability to induce granuloma and recruit natural killer T cells. J. Biol. Chem. 276: 34896-34904.
Kates, M (1986) Techniques of lipidology: isolation, analysis and identification of lipids. In Laboratory techniques in biochemistry and molecular biology. Vol. 3. Part 2. Edited by R.H. Burdon and P.H. van Knippenberg. Elsevier, New York.
Koike, Y., Y.C. Yoo, M. Mitobe, T. Oka, K. Okuma, S. Tono-oka, and 1. Azuma (1998) Enhancing activity of mycobacterial cell-derived adjuvants on immunogenicity of recombinant human hepatitis B virus vaccine. Vaccine 16: 1982-1989.
Krishnan, L., C.J. Dicaire, G.B. Patel, and G.D. Sprott (2000) Archaeosome vaccine adjuvants induce strong humoral, cell-mediated, and memory responses: Comparison to conventional liposomes and alum. Infection and Immunity 68: 54-63.
Krishnan, L., S. Sad, G.B. Patel, and G.D. Sprott (2001) Archaeosomes induce long-term CD8+ cytotoxic T cell response to entrapped soluble protein by the exogenous cytosolic pathway, in the absence of CD4+ T cell help. J. Immunol. 165: 5177-5185
Leopold, K. and W. Fischer (1993) Molecular analysis of the lipoglycans of Mycobacterium tuberculosis. Analytical Biochemstry 208: 57-64.
Mára, M., J. Julák, E. Men 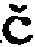 iková, J. O 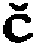 ená 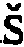 ková, and A. Dohnalová (1992) Effect ofcrude bacterial lipids on the course of *Listeria* infection in mice. Folia Microbiol. 37: 455-460.
Means, T.K., E. Lien, A. Yoshimura, S. Wang, D.T. Golenbock, and M.J. Fenton (1999) The CD14 ligands lipoarabinomannan and lipopolysaccharide differ in their requirement for Toll-like receptors. J. Immunol. 163: 6748-6755.
Mosmann, T.R., and T.A. Fong (1989) Specific assays for cytokine production by T cells. J. Immunol. Methods 116: 151-158.
Nigou, J., M. Gilleron, B. Cahuzac, J.-D. Bounéry, M. Herold, M. Thurnhert, and G. Puzo (1997) The phosphatidyl-myo-inositol anchor of the lipoarabinomannans from Mycobacterium bovis Bacillus Calmette Guérin. J. Biol. Chem. 272: 23094-23103.
Ortalo-Magné, A., A. Lemassu, M.-A. Lanéelle, F. Bardou, G. Silve, P. Gounon, G. Marchal, and M. Daffé (1996) Identification of the surface-exposed lipids on the cell envelopes of Mycobacterium tuberculosis and other mycobacterial species. J. Bacteriol. 178:456-461.
Retzinger, G.S., S.C. Meredith, K. Takayama, R.L. Hunter, and F.J. Kézdy (1981) The role of surface in the biological activities of trehalose 6,6'-dimycolate. J. Biol. Chem. 256: 8208-8216.
Skinner, M.A., R. Prestidge, S. Yuan, T.J. Strabala, and P.L.J. Tan (2001) The ability of heat-killed Mycobacterium vaccae to stimulate a cytotoxic T-cell response to an unrelated protein is associated with a 65 kilodalton heat-shock protein. Immunology 102: 225-233.
Sprott, G.D., C.G. Choquet, and G.B. Patel (1995) Purification of ether lipids and liposome formation from polar lipid extracts of methanogenic bacteria. In F.T. Robb et al. (Eds.), Archaea: A laboratory manual. Cold Spring Harbor Laboratory Press, Cold Spring Harbor, NY, pp. 329-340.
Vijh, S. and Pamer, E.G. (1997) Immunodominant and subdominant CTL responses to Listeria monocytogenes infection. J. Immunol. 158: 3366-3371.
Wang, L., R.A. Slayden, C.E. Barry III, and J. Liu (2000) Cell wall structure of a mutant of Mycobacterium smegmatis defective in the biosynthesis of mycolic acids. J. Biol. Chem. 275: 7224-7229.

## Claims

1. A liposome comprising one or more lipids obtainable from a mycobacterium species, and selected from phosphatidylinositol (PI), phosphatidylinositol mannoside (PIM₁), phosphatidylinositol dimannoside (PIM₂), mono and dipalmitoylated forms of PIM₁ and PIM₂, acylated-phospholipids having fast atom bombardment mass spectrometry peaks at 899, 1139 and 1155 m/z, phosphatidyl-ethanolamine and cardiolipid, wherein said lipids that are obtainable from mycobacterium are chloroform soluble and extractable.

2. A liposome according to claim 1, wherein the mycobacterium species is *Mycobacterium bovis* BCG.

3. A liposome according to either one of claims 1 or 2, wherein the chloroform soluble and extractable lipid is PTM₁.

4. A liposome according to either one of claims 1 or 2, wherein the chloroform soluble and extractable lipid is PIM₂.

5. A liposome according to either one of claims 1 or 2, wherein the chloroform soluble and extractable lipid is palmitoyl-PIM₁.

6. A liposome according to either one of claims 1 or 2, wherein the chloroform soluble and extractable lipid is palmitoyl-PIM₂.

7. A liposome according either one of claims 1 or 2, wherein the chloroform soluble and extractable polar lipid is obtainable by a hot 50% ethanol extraction.

8. A liposome according to either one of claims 1 or 2, comprising lipid phosphatidylethanolamine.

9. A liposome according to claims 1 to 2 further comprising at least one other lipid, wherein the other lipid is phosphatidylcholine, phosphatidylglycerol, cholesterol, or a mixture of any of these.

10. A liposome vaccine composition comprising a liposome according to any one of claims 1 to 9, wherein the liposome contains an associated antigen.

11. A liposome vaccine composition according to claim 10, wherein the antigen is a protein.

12. A method for preparing a liposome according to any one of claims 1 to 9, which method comprises drying chloroform soluble and extractable lipid from Mycobacterium SPP and then hydrating said dried lipid at a temperature of 65 to 75°C in water or phosphate buffered saline (PBS).

13. A method according to claim 12, wherein said temperature is 65°C.

14. A method according to claim 12, wherein said liposome resulting from said method is multilamellar.

15. A method according to claim 14, additionally comprising reducing the size of a multilamellar liposome at a temperature of 65°C to yield a unilamellar liposome.

16. A method according to claim 14, wherein an antigen is entrapped in said multilamellar liposome by inclusion of said antigen in said water or phosphate buffered saline.

17. A method according to claim 16, wherein an antigen is entrapped in said unilamellar liposome by inclusion of said antigen in said water or phosphate buffered saline.

18. Use of a liposome according to any one of claims 1 to 9, in the manufacture of a medicament for stimulating an immune response in a mammal.

19. Use according to Claim 18, wherein stimulating the immune response in a mammal is achieved by activating dendritic cells wherein activation elicits secretion of Interleukin-12 (IL-12) by said dendritic cells.

20. Use according to Claim 18, wherein stimulating the immune response in a mammal is achieved by activating dendritic cells wherein activation elicits secretion of tumour necrosis factor (TNF) by said dendritic cells.

21. Use according to Claim 18, wherein stimulating the immune response in a mammal is achieved by activating dendritic cells wherein activation elicits secretion of Interleukin-12 (IL-12) and tumour necrosis factor (TNF) by said dendritic cells.

22. The use of a liposome according to any one of claims 18 to 21, wherein said liposome additionally comprises an antigen.

23. Use of a liposome vaccine composition according to claim 10 or 11, in preparing a medicament to elicit an immune response in a mammal.

24. The use according to claim 23, wherein the liposome serves both as a carrier for the antigen and as a stimulator of an immune response.

25. The use according to claim 23, wherein the immune response is an antigen specific antibody response and an antigen specific cytotoxic T cell response.

26. Use of a liposome vaccine composition according to claim 10 or 11, in preparing a medicament for the therapeutic or prophylactic treatment of cancer.

27. Use of a liposome vaccine composition according to claim 10 or 11, in preparing a medicament to direct an immune response to confer protection against a pathogen or a cancer.

## Patentansprüche

1. Ein Liposom, das ein oder mehrere aus einer Gattung Mycobacterium erhältliche Lipide beinhaltet, die aus Phosphatidylinositol (PI), Phosphatidylinositolmannosid (PIM₁), Phosphatidylinositoldimannosid (PIM₂), mono- und dipalmitoylierten Formen von PIM₁ und PIM₂, acylierten Phospholipiden mit Fast-Atom-Bombardment-Massenspektrometriespitzen bei 899, 1139 und 1155 m/q, Phosphatidylethanolamin und Cardiolipid ausgewählt sind,
wobei die aus Mycobacterium erhältlichen Lipide in Chloroform löslich und damit extrahierbar sind.

2. Liposom gemäß Anspruch 1, wobei die Gattung Mycobacterium *Mycobacterium bovis* BCG ist.

3. Liposom gemäß einem der Ansprüche 1 oder 2,
wobei das in Chloroform lösliche und damit extrahierbare Lipid PIM₁ ist.

4. Liposom gemäß einem der Ansprüche 1 oder 2,
wobei das in Chloroform lösliche und damit extrahierbare Lipid PIM₂ ist.

5. Liposom gemäß einem der Ansprüche 1 oder 2, wobei das in Chloroform lösliche und damit extrahierbare Lipid Palmitoyl-PIM₁ ist.

6. Liposom gemäß einem der Ansprüche 1 oder 2, wobei das in Chloroform lösliche und damit extrahierbare Lipid Palmitoyl-PIM₂ ist.

7. Liposom gemäß einem der Ansprüche 1 oder 2, wobei das in Chloroform lösliche und damit extrahierbare polare Lipid durch eine Extraktion mit 50%igem heißen Ethanol erhältlich ist.

8. Liposom gemäß einem der Ansprüche 1 oder 2, das Lipidphosphatidylethanolamin beinhaltet.

9. Liposom gemäß den Ansprüchen 1 bis 2, das ferner mindestens ein anderes Lipid beinhaltet, wobei das andere Lipid Phosphatidylcholin, Phosphatidylglycerin, Cholesterin oder eine Mischung von beliebigen aus diesen ist.

10. Eine Liposomimpfstoffzusammensetzung, die ein Liposom gemäß einem der Ansprüche 1 bis 9 beinhaltet, wobei das Liposom ein assoziiertes Antigen enthält.

11. Liposomimpfstoffzusammensetzung gemäß Anspruch 10,
wobei das Antigen ein Protein ist.

12. Ein Verfahren zum Zubereiten eines Liposoms gemäß einem der Ansprüche 1 bis 9, wobei das Verfahren das Trocknen von in Chloroform löslichem und damit extrahierbarem Lipid aus Mycobacterium SPP und dann das Hydratisieren des getrockneten Lipids bei einer Temperatur von 65 bis 75 °C in Wasser oder phosphatgepufferter Salzlösung (PBS) beinhaltet.

13. Verfahren gemäß Anspruch 12, wobei die Temperatur 65 °C beträgt.

14. Verfahren gemäß Anspruch 12, wobei das aus dem Verfahren resultierende Liposom multilamellar ist.

15. Verfahren gemäß Anspruch 14, das zusätzlich das Reduzieren der Größe eines multilamellaren Liposoms bei einer Temperatur von 65 °C beinhaltet, um ein unilamellares Liposom zu ergeben.

16. Verfahren gemäß Anspruch 14, wobei ein Antigen durch Inklusion des Antigens in dem Wasser oder der phosphatgepufferten Salzlösung (PBS) in dem multilamellaren Liposom eingeschlossen ist.

17. Verfahren gemäß Anspruch 16, wobei ein Antigen durch Inklusion des Antigens in dem Wasser oder der phosphatgepufferten Salzlösung (PBS) in dem unilamellaren Liposom eingeschlossen ist.

18. Eine Verwendung eines Liposoms gemäß einem der Ansprüche 1 bis 9 bei der Erzeugung eines Medikaments zur Anregung einer Immunreaktion bei einem Säugetier.

19. Verwendung gemäß Anspruch 18, wobei die Anregung der Immunreaktion bei einem Säugetier durch die Aktivierung dendritischer Zellen erreicht wird, wobei die Aktivierung die Absonderung von Interleukin-12 (IL-12) durch die dendritischen Zellen auslöst.

20. Verwendung gemäß Anspruch 18, wobei die Anregung der Immunreaktion bei einem Säugetier durch die Aktivierung dendritischer Zellen erreicht wird, wobei die Aktivierung die Absonderung von Tumornekrosefaktor (TNF) durch die dendritischen Zellen auslöst.

21. Verwendung gemäß Anspruch 18, wobei die Anregung der Immunreaktion bei einem Säugetier durch die Aktivierung dendritischer Zellen erreicht wird, wobei die Aktivierung die Absonderung von Interleukin-12 (IL-12) und Tumornekrosefaktor (TNF) durch die dendritischen Zellen auslöst.

22. Verwendung eines Liposoms gemäß einem der Ansprüche 18 bis 21, wobei das Liposom zusätzlich ein Antigen beinhaltet.

23. Verwendung einer Liposomimpfstoffzusammensetzung gemäß Anspruch 10 oder 11 beim Zubereiten eines Medikaments zum Auslösen einer Immunreaktion bei einem Säugetier.

24. Verwendung gemäß Anspruch 23, wobei das Liposom sowohl als ein Träger für das Antigen als auch als ein Stimulator einer Immunreaktion dient.

25. Verwendung gemäß Anspruch 23, wobei die Immunreaktion eine antigenspezifische Antikörperreaktion und eine antigenspezifische zytotoxische T-Zell-Reaktion ist.

26. Verwendung einer Liposomimpfstoffzusammensetzung gemäß Anspruch 10 oder 11 beim Zubereiten eines Medikaments zur therapeutischen oder prophylaktischen Behandlung von Krebs.

27. Verwendung einer Liposomimpfstoffzusammensetzung gemäß Anspruch 10 oder 11 beim Zubereiten eines Medikaments zur Anleitung einer Immunreaktion, um einen Schutz gegen ein Pathogen oder Krebs zu verleihen.

## Revendications

1. Un liposome comprenant un ou plusieurs lipides qui peuvent être obtenus à partir d'une espèce de mycobactérie, et qui sont sélectionnés parmi le phosphatidylinositol (PI), le phosphatidylinositol mannoside (PIM₁), le phosphatidylinositol dimannoside (PIM₂), des formes mono et dipalmitoylées de PIM₁ et PIM₂, des phospholipides acylés ayant des pics de spectrométrie de masse par bombardement avec des atomes rapides à 899,1139 et 1155 m/z, la phosphatidyl éthanolamine et le cardiolipide, dans lequel lesdits lipides qui peuvent être obtenus à partir d'une mycobactérie sont solubles dans et extractibles par le chloroforme.

2. Un liposome selon la revendication 1, dans lequel l'espèce de mycobactérie est le *Mycobacterium bovis* BCG.

3. Un liposome selon l'une ou l'autre des revendications 1 et 2, dans lequel le lipide soluble dans et extractible par le chloroforme est le PIM₁.

4. Un liposome selon l'une ou l'autre des revendications 1 et 2, dans lequel le lipide soluble dans et extractible par le chloroforme est le PIM₂.

5. Un liposome selon l'une ou l'autre des revendications 1 et 2, dans lequel le lipide soluble dans et extractible par le chloroforme est le palmitoyl-PIM₁.

6. Un liposome selon l'une ou l'autre des revendications 1 et 2, dans lequel le lipide soluble dans et extractible par le chloroforme est le palmitoyl-PIM₂.

7. Un liposome selon l'une ou l'autre des revendications 1 et 2, dans lequel le lipide polaire soluble dans et extractible par le chloroforme peut être obtenu par une extraction avec 50 % d'éthanol chaud.

8. Un liposome selon l'une ou l'autre des revendications 1 et 2, comprenant le lipide phosphatidyléthanolamine.

9. Un liposome selon les revendications 1 et 2 comprenant en outre au moins un autre lipide, dans lequel l'autre lipide est la phosphatidylcholine, le phosphatidylglycérol, le cholestérol, ou un mélange de n'importe lesquels de ceux-ci.

10. Une composition de vaccin à liposome comprenant un liposome selon l'une quelconque des revendications 1 à 9, dans laquelle le liposome contient un antigène associé.

11. Une composition de vaccin à liposome selon la revendication 10, dans laquelle l'antigène est une protéine.

12. Un procédé pour préparer un liposome selon n'importe laquelle des revendications 1 à 9, lequel procédé comprend faire sécher le lipide soluble dans et extractible par le chloroforme provenant de Mycobacterium SPP, puis hydrater ledit lipide séché à une température d'entre 65 et 75 °C dans de l'eau ou une solution salée tamponnée au phosphate (PBS).

13. Un procédé selon la revendication 12, dans lequel ladite température est de 65 °C.

14. Un procédé selon la revendication 12, dans lequel ledit liposome résultant dudit procédé est multilamellaire.

15. Un procédé selon la revendication 14, comprenant de manière additionnelle faire réduire la taille d'un liposome multilamellaire à une température de 65 °C pour donner un liposome unilamellaire.

16. Un procédé selon la revendication 14, dans lequel un antigène est emprisonné dans ledit liposome multilamellaire par inclusion dudit antigène dans ladite eau ou solution salée tamponnée au phosphate.

17. Un procédé selon la revendication 16, dans lequel un antigène est emprisonné dans ledit liposome unilamellaire par inclusion dudit antigène dans ladite eau ou solution salée tamponnée au phosphate.

18. Utilisation d'un liposome selon l'une quelconque des revendications 1 à 9, dans la fabrication d'un médicament pour stimuler une réponse immunitaire chez un mammifère.

19. Utilisation selon la revendication 18, dans laquelle la stimulation de la réponse immunitaire chez un mammifère est obtenue en activant des cellules dendritiques où l'activation élicite la sécrétion d'lnterleukine-12 (IL-12) par lesdites cellules dendritiques.

20. Utilisation selon la revendication 18, dans laquelle la stimulation de la réponse immunitaire chez un mammifère est obtenue en activant des cellules dendritiques où l'activation élicite la sécrétion de facteur de nécrose tumorale (TNF) par lesdites cellules dendritiques.

21. Utilisation selon la revendication 18, dans laquelle la stimulation de la réponse immunitaire chez un mammifère est obtenue en activant des cellules dendritiques où l'activation élicite la sécrétion d'lnterleukine-12 (IL-12) et de facteur de nécrose tumorale (TNF) par lesdites cellules dendritiques.

22. L'utilisation d'un liposome selon l'une quelconque des revendications 18 à 21, dans laquelle ledit liposome comprend de manière additionnelle un antigène.

23. Utilisation d'une composition de vaccin à liposome selon la revendication 10 ou la revendication 11, dans la préparation d'un médicament pour éliciter une réponse immunitaire chez un mammifère.

24. L'utilisation selon la revendication 23, dans laquelle le liposome sert à la fois d'excipient pour l'antigène et de stimulateur d'une réponse immunitaire.

25. L'utilisation selon la revendication 23, dans laquelle la réponse immunitaire est une réponse anticorps spécifique de l'antigène et une réponse T cytotoxique spécifique de l'antigène.

26. Utilisation d'une composition de vaccin à liposome selon la revendication 10 ou la revendication 11, dans la préparation d'un médicament destiné au traitement thérapeutique ou prophylactique du cancer.

27. Utilisation d'une composition de vaccin à liposome selon la revendication 10 ou la revendication 11, dans la préparation d'un médicament afin de diriger une réponse immunitaire de manière à conférer une protection contre un pathogène ou un cancer.
